(19) Europäisches Patentamt
European Patent Office
Office européen des brevets

(11) Publication number : **0 397 452 B1**

(12) **EUROPEAN PATENT SPECIFICATION**

(45) Date of publication of patent specification : **29.09.93 Bulletin 93/39**

(51) Int. Cl.$^5$ : **A61K 7/16**

(21) Application number : **90304950.0**

(22) Date of filing : **08.05.90**

(54) **Anti-calculus toothpaste compositions having improved shelf-life.**

(30) Priority : **08.05.89 US 348805**
**14.09.89 US 407336**
**29.03.90 GB 9007074**

(43) Date of publication of application :
**14.11.90 Bulletin 90/46**

(45) Publication of the grant of the patent :
**29.09.93 Bulletin 93/39**

(84) Designated Contracting States :
**AT DE DK ES LU NL SE**

(56) References cited :
**GB-A- 2 200 551**
**US-A- 4 627 977**
**US-A- 4 806 342**
**US-A- 4 808 400**

(73) Proprietor : **Beecham Inc.**
**One Franklin Plaza**
**Philadelphia, PA 19101 (US)**

(72) Inventor : **Ibrahim, Nader**
**Beecham Products, 1500 Littleton road**
**Parsippany, New Jersey 07054 (US)**
Inventor : **Sodano, Jeanette L.**
**Beecham Products, 1500 Littleton road**
**Parsippany, New Jersey 07054 (US)**

(74) Representative : **Connell, Anthony Christopher et al**
**SmithKline Beecham Corporate Patents Great Burgh Yew Tree Bottom Road**
**Epsom, Surrey KT18 5XQ (GB)**

EP 0 397 452 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to oral hygiene compositions, in particular compositions comprising a tripolyphosphate salt, which compositions are useful in the inhibition of dental calculus.

Several proposals for oral hygiene compositions comprising a tripolyphosphate salt as an anti-calculus agent have been made. For instance, EP-A-0 236 290 (to Monsanto) discloses dentifrices comprising inter alia a tripolyphosphate salt in combination with an abrasive which is calcium pyrophosphate, the pH of which is preferably less than 8. EP-A-0 295 116 (to Unilever) discloses anti-calculus compositions comprising a combination of a tripolyphosphate salt and zinc citrate as the anti-calculus agent whilst EP-A-0 254 452 (to Lion Corporation) discloses anti-calculus compositions comprising a combination of a tripolyphosphate salt and anethol as the anti-calculus agent. Each of the two latter applications describes an example comprising a silica abrasive, with sodium tripolyphosphate included at 5% and 1.5% by weight of the composition, respectively. In neither case is any teaching provided with respect to the pH of the composition.

US 4 627 977 (to Colgate-Palmolive) discloses anti-calculus compositions comprising a linear molecularly dehydrated polyphosphate salt such as a tripolyphosphate in which the otherwise observed enzymatic hydrolysis of the anti-calculus agent by phosphatase enzymes present in saliva is reduced by including a mixture of a source of fluoride ions and a synthetic anionic linear polymeric carboxylate. The preferred pH of such compositions is in the rante 5.5 to 8. The patent includes an example comprising sodium tripolyphosphate at 5% by weight of the composition.

In addition, GB-A-2 200 551 (to Colgate-Palmolive) discloses compositions having anti-calculus and anti-plaque activity, the former being provided by a linear molecularly dehydrated polyphosphate salt which is preferably a pyrophosphate salt, and the latter being provided by a non-cationic anti-bacterial agent such as a hydroxydiphenylether, and preferably triclosan. The preferred pH for such compositions is in the range 5.5 to 8. No examples comprising a tripolyphosphate are provided.

In spite of the various proposals referred to above, as far as we are aware, no anti-calculus dentifrice comprising a tripolyphosphate salt such as sodium tripolyphosphate has yet been marketed. One particular problem to be overcome in developing a commercially viable composition is that of the storage stability of the composition.

This problem is addressed in EP-A-0 236 290. This application provides storage stability data on two toothpastes comprising a calcium polyphosphate abrasive and sodium tripolyphosphate at 5% by weight of the composition, but which differ in having pHs of 8.5 and 7.1. Under accelerated aging conditions, both toothpastes suffer a similar loss of about one third in the amount of sodium tripolyphosphate present, suggesting that a higher pH per se is not a critical factor. This loss is considered to be unsatisfactory for a commercial product. JP-A-50161/88 (to Lion Corporation) suggests that storage stability may be improved by selecting as the abrasive certain silicas characterised by their surface area. There is however no discussion of the influence of pH or the amount of tripolyphosphate salt on storage stability. No storage stability data is provided in the other patent applications or patents referred to above.

It has now been found that the problem of storage stability may be remedied by providing a relatively large amount of the tripolyphosphate salt at a high pH, contrary to the teachings of the prior art.

Accordingly, in a first aspect, the present invention provides a toothpaste composition which comprises: an anti-calculus agent comprising at least 4% by weight, based on the total weight of the toothpaste, of a water-soluble alkali metal tripolyphosphate salt; a dental abrasive other than calcium pyrophosphate; and an orally acceptable vehicle; the toothpaste having a pH of from 8 to 10.

Such a toothpaste is found to have sufficient storage stability for a commercially viable product.

It has been further found that an oral hygiene composition may be provided having, in addition to anti-calculus activity, anti-plaque activity by incorporating, in addition to a tripolyphosphate salt, an anti-bacterial compound.

Accordingly, in a second aspect of the invention, there is provided a toothpaste composition as described above further comprising a substantially water insoluble noncationic anti-bacterial agent.

Suitable noncationic anti-bacterial agents include for example diphenyl ethers of the formula (I):

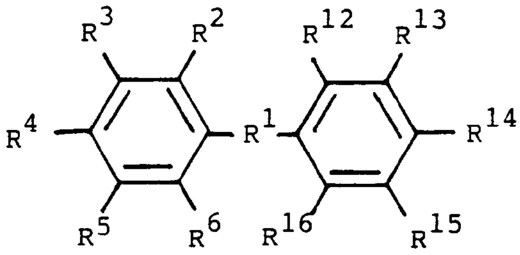

(I)

in which $R^1$ is oxygen, sulphur, or an alkylene group of from one to six carbon atoms and each of $R^2$ to $R^6$ and $R^{12}$ to $R^{16}$ is hydrogen, hydroxyl or a halogen; phenolic and bisphenolic compounds; benzoate esters and halogenated carbanilides

Examples of compounds of formula (I) include, for example, 5,5'-dichloro-2,2'-dihydroxydiphenylmethane; 2,2'-dihydroxy-3,5,6,3',5',6'-hexachlorodiphenylmethane; 3,3'-dibromo-5,5'-dichloro-2,2'-dihydroxydiphenylether and 2,4,4'-trichloro-2'-hydroxydiphenyl ether (triclosan); of which triclosan is particularly preferred.

Examples of phenolic compounds, which include the halogenated salicylanilides, include, for example,
2-phenylphenol,
4-chlorophenol,
4-chloro-3-methylphenol,
4-chloro-3-methylphenol,
4-chloro-3,5-dimethylphenol,
2,4-dichloro-3,5-dimethylphenol,
5-methyl-2-pentylphenol,
4-isopropyl-3-methylphenol,
5-chloro-2-hydroxydiphenylmethane,
4',5-dibromosalicylanilide,
3,4',5-tribromosalicylanilide,
2,3,3',5-tetrachlorosalicylanilide,
3,3',4,5'-tetrachlorosalicylanilide,
3,5-dibromo-3'-trifluoromethylsalicylanilide, and
5-n-octanoyl-3'-trifluoromethylsalicylanilide

Examples of bisphenolic compounds include, for example,
2,2'-methylenebis(3,4,6-trichlorophenol),
2,2'-methylenebis(4-chlorophenol),
2,2'-methylenebis(4-chloro-6-bromophenol),
bis(2-hydroxy-3,5-dichlorophenyl)sulphide, and
bis(2-hydroxy-5-chlorophenyl)sulphide.

Examples of benzoate esters include, for example, esters of hydroxybenzoic acid, especially the methyl, ethyl, propyl, isopropyl, butyl, isobutyl, hexyl, heptyl and benzyl esters.

Examples of halogenated carbanilides include, for example,
3,4,4'-trichlorocarbanilide,
3-trifluoromethyl-4,4'-dichlorocarbanilide, and
3,3',4-trichlorocarbanilide.

The preferred noncationic anti-bacterial agents are compounds of formula (I), in particular the compound triclosan.

The noncationic anti-bacterial agent is preferably present in from 0.01 to 2.0%, more preferably 0.05 to 1.0% by weight of the composition.

It will be appreciated that the alkali metal tripolyphosphate salt is included in compositions of the invention as an anti-calculus agent. In some instances, the anti-calculus agent may consist essentially of the water-soluble alkali metal tripolyphosphate salt.

Suitable water-soluble alkali metal salts include sodium or potassium tripolyphosphate which may be used in the hydrated or unhydrated forms. Preferably the tripolyphosphate salt is present in at least 4%, more preferably from 4 to 6% by weight, based on the total weight of the composition.

The pH of the composition is from 8 to 10, more preferably from 8 to 9. When the composition comprises a compound of formula (I), a pH of above 8 is found to enhance the availability of the compound of formula

(I). All references to the pH of the composition herein are references to the pH of the composition measured without dilution of the composition.

Suitable abrasives for use in the present invention include silica, plastics particles, alumina, calcium carbonate, and zinc orthophosphate, and if a noncationic antibacterial agent as hereinbefore defined is present, calcium pyrophosphate. Silica is especially preferred.

Silica abrasives are well known and commercially available, generally having an average particle size ranging between 0.1 to 30 μm (microns), such as from 5 to 15 μm (microns). Silica dental abrasives useful in the present invention include those marketed by the J.M. Huber Corporation under the trade name 'Zeodent' and the silica zerogels marketed by the W.R. Grace and Company, Davison Chemical Division under the trade name 'Syloid'. US 3 358 230 and US 3 862 307 describe silica dental abrasives that are useful in the toothpaste compositions according to the present invention. The silica abrasive may also be a naturally occurring amorphous silica such as diatomaceous earth. Suitable forms of diatomaceous earth are those marketed under the trade mark 'Celite' by Johns - Manville Products Corporation, for instance 'Celite Superfine Superfloss'.

Plastics dental abrasives are well known and are described in, for example, GB 939 230, GB 995 351 and GB 1 055 784, and US 3 151 027.

Alumina abrasives are well known and commercially available. Preferably the alumina abrasive may be treated with a solution of a surface-treating agent which may be an alkali metal silicate, hydrogen peroxide, an acid or an organophosphorus compound, of which an alkali metal silicate is especially preferred, as described in US 4 781 982 (to Aluminium Company of America).

A calcium carbonate abrasive is preferably used in conjunction with an ionic agent to suppress the formation of free calcium ions, such as an alkali metal carbonate or bicarbonate, or mixture thereof, as described in EP 0 092 929 (to Beecham Group p.l.c.).

Generally, an amount of the dental abrasive suitable for use in the toothpaste composition of the present invention will be empirically determined to provide an acceptable level of cleaning and polishing, in accordance with the techniques well known in the art. Suitably, the abrasive will be present in from 5 to 60%, preferably from 5 to 30%, by weight of the toothpaste.

Advantageously, compositions of the present invention may further comprise a phosphatase enzyme inhibitor comprising a fluoride ion source, to optimise the anti-calculus activity of the compositions by inhibiting the enzymatic hydrolysis of the tripolyphosphate salt by salivary phosphatase enzymes. The fluoride ion source may be provided by an alkali metal fluoride, preferably sodium fluoride, an alkali metal monofluorophosphate, stannous fluoride and the like. Preferably, however, the fluoride ion source is an alkali metal fluoride, most preferably sodium fluoride, since this appears to provide enhanced storage stability as compared to other fluoride ion sources. The fluoride ion source serves as a phosphatase enzyme inhibitor, and in addition, the fluoride ion source may also provide an anti-caries effect. Preferably, the fluoride ion source will be used in an amount to provide an anti-caries effective amount and a phosphatase enzyme inhibiting amount, such as an amount sufficient to provide from 25 ppm to 3500 ppm, preferably 1100 ppm, fluoride. It will be appreciated that the fluoride ion source may be included in compositions of the invention to provide a phosphatase enzyme inhibitor. In some instances, the phosphatase enzyme inhibitor may consist essentially of a fluoride ion source.

Suitably, in compositions of the present invention, the orally acceptable vehicle may comprise a thickening agent, a binding agent and a humectant. Preferred thickening and binding agents include for example natural and synthetic gums such as xanthan gums, carageens, alginates, cellulose ethers and esters and silica. When the abrasive is silica, it is preferred to use a thickening silica as the thickening agent. Preferred humectants include glycerin, sorbitol, propylene glycol and polyethylene glycol. A preferred humectant system consists of glycerin, sorbitol and polyethylene glycol.

In addition, the orally acceptable vehicle may optionally comprise surfactants, sweetening agents, flavouring agents, anticaries agents (in addition to the fluoride ion source provided as a phosphatase enzyme inhibitor), anti-plaque agents, anti-bacterial agents such as triclosan or cetyl pyridinium chloride, tooth desensitizing agents, colouring agents and pigments. Useful surfactants include the water-soluble salts of alkyl sulphates having from 10 to 18 carbon atoms in the alkyl moiety, such as sodium lauryl sulphate, but other anionic surfactants as well as non-ionic, zwitter-ionic, cationic and amphoteric surfactants may also be used.

When the preferred aqueous orally acceptable dental vehicle is employed, a toothpaste composition of the present invention suitably contains from 10 to 80% humectant, from 0.25 to 5% detergent, from 0 to 2% sweeteners and flavouring agents together with water and an effective amount of binding and thickening agents, such as from 0.1% to 12%, to provide the toothpaste of the invention with the desired stability and flow characteristics.

Toothpaste compositions according to the present invention may be prepared by admixing according to conventional practice the tripolyphosphate salt, the dental abrasive and, if included, the fluoride ion source with the orally acceptable dental vehicle, which may be anhydrous but is preferably an aqueous orally acceptable dental vehicle, to form a storage stable semi-solid extrudable material useful as a toothpaste The pH thereof may be adjusted if necessary and desired, by the addition of, for instance sodium hydroxide. Preferably, the tripolyphosphate salt is in powder form when incorporated into the vehicle, as this tends to enhance the stability of the resulting toothpaste.

Toothpaste compositions of the present invention may also be prepared in the form of a paste of a uniform colour or in the form of a striped toothpaste. A suitable aparatus for filling toothpaste tubes with striped toothpaste is described in GB 962 757. In accordance with this patent, toothpastes of different colours are fed through separate tubes of a bundle of tubes that is inserted into a toothpaste container and gradually moved relative to the container as the container is filled.

In a third aspect, the present invention provides a method of inhibiting dental calculus which method comprises applying an anti-calculus effective amount of a composition as hereinbefore defined to the oral cavity; and optionally a method of also inhibiting dental plaque, when a compound of formula (I) is also present in the composition.

The invention will now be illustrated by the following examples:

## Example 1

A toothpaste was prepared from the following ingredients, sodium tripolyposphate being in powder form when combined with the other ingredients:

| | | % w/w |
|---|---|---|
| Polyethylene glycol (PEG-8) | | 3.00 |
| Xanthan Gum | | 0.70 |
| D + C Red No. 30 Lake | | 0.02 |
| FD + C Blue No. 1 (0.2%) | | 0.17 |
| D + C Yellow No. 10 (0.20%) | | 0.24 |
| Sodium Fluoride | | 0.24 |
| Sorbitol (70%) | | 29.61 |
| Sodium Saccharin | | 0.21 |
| Thickening Silica | | 8.00 |
| Abrasive Silica | | 14.00 |
| Titanium Dioxide | | 0.96 |
| Glycerine 99% | | 10.00 |
| Sodium Tripolyphosphate (Food Grade)* | | 5.00 |
| Sodium Hydroxide (25%) | | 1.80 |
| Flavour | | 0.80 |
| Sodium Lauryl Sulphate | | 1.15 |
| Deionized Water | to | 100.00 |

* Food grade has a nominal purity of 92%.

Initial pH 8.4

Examples 2 - 5 - Striped dentifrices

The preparation of striped dentrifrices is well known in the art. US Patent Nos. 3 996 863, 3 980 767, 4 328 205 and 4 358 437 described toothpastes and methods for production thereof which may be utilised for the production of the dentifrices according to the present invention.

Example 2

A striped dentifrice according to the present invention and comprising a central core with red stripes and aqua stripes was prepared by combining the ingredients set forth below according to known conventional techniques.

| | % w/w | | |
|---|---|---|---|
| | Core | Red | Aqua |
| PEG* 400 | 3.00 | 3.00 | 3.00 |
| Xanthan Gum | 0.70 | 0.70 | 0.70 |
| Sodium Fluoride | 0.24 | 0.24 | 0.24 |
| Sodium Saccharin | 0.21 | 0.21 | 0.21 |
| Triclosan | 0.20 | 0.20 | 0.20 |
| Sorbitol (70%) | 29.09 | 29.09 | 29.09 |
| FD + C Blue No.1 dye | - | - | 0.0020 |
| D + C Yellow No. 10 dye | - | - | 0.0002 |
| Titanium Dioxide | 1.459 | - | - |
| Abrasive Silica | 14.00 | 14.00 | 14.00 |
| Thickening Silica | 8.00 | 8.00 | 8.00 |
| Sodium Tripolyphosphate | 5.00 | 5.00 | 5.00 |
| Glycerine | 10.00 | 10.00 | 10.00 |
| NaOH | 0.45 | 0.45 | 0.45 |
| Flavour | 0.80 | 0.80 | 0.80 |
| Sodium Lauryl Sulphate | 1.15 | 1.15 | 1.15 |
| Deionized Water to | 100.00 | 100.00 | 100.00 |

*PEG denotes polyethylene glycol.

Example 3

As Example 2 but with triclosan at 0.3%.

Example 4

| | % w/w | | |
|---|---|---|---|
| | Core | Red | Aqua |
| PEG* 300 | 3.00 | 3.00 | 3.00 |
| Xanthan Gum | 0.60 | 0.60 | 0.60 |
| Sodium Fluoride | 0.22 | 0.22 | 0.22 |
| Sodium Saccharin | 0.20 | 0.20 | 0.20 |
| Triclosan | 0.20 | 0.20 | 0.20 |
| Sorbitol (70%) | 27.70 | 27.70 | 27.70 |
| FD + C Blue No.1 dye | - | - | 0.002 |
| D + C Yellow No. 10 dye | - | - | 0.001 |
| D + C Yellow No. 3 dye | - | 0.10 | - |
| Titanium Dioxide | 1.45 | - | - |
| Abrasive Silica | 14.00 | 14.00 | 14.00 |
| Thickening Silica | 7.00 | 7.00 | 7.00 |
| Sodium Tripolyphosphate | 5.00 | 5.00 | 5.00 |
| Glycerine | 20.00 | 20.00 | 20.00 |
| Flavour | 0.80 | 0.80 | 0.80 |
| Sodium Lauryl Sulphate | 1.70 | 1.70 | 1.70 |
| Deionized Water to | 100.00 | 100.00 | 100.00 |

*PEG denotes polyethylene glycol.

Example 5

As Example 4 but with triclosan at 0.3%.

Example 6

Toothpastes A (as per Example 1), B, C, and D were prepared using a silica dental abrasive, sodium tripolyphosphate (STP), and sodium fluoride in an amount to provide 1100 ppm fluoride, in an aqueous orally acceptable vehicle. The amounts of STP and the pH of the toothpastes were varied as shown in the Table below:

Table

| Tooth-paste | pH* | STP% | % STP As % Of Initial STP After | | | |
|---|---|---|---|---|---|---|
| | | | 1 year | 2 year | 3 year | 5 year |
| A | 9 | 4.3 | 98 | 96 | 94 | 91 |
| B | 7 | 4.3 | 96 | 93 | 89 | 85 |
| C | 9 | 3.0 | 90 | 80 | 69 | 59 |
| D | 7 | 2.8 | 85 | 70 | 55 | 39 |

*The pH's reported are nominal values.

The data presented in the Table were derived from storage stability tests on actual samples of the four toothpastes, with the storage stabilty data regressed to provide predicted stability over four years of storage at room temperature with a confidence level of 95%.

As can be seen from the table, toothpaste A which contains a large amount of STP and has a high pH is the most stable of the four toothpastes.

In comparison, either a large amount of STP (B) alone or a high pH (C) alone provides inferior storage stability.

Unexpectedly, the use of a large amount of STP and a high pH in combination with a silica dental abrasive and a fluoride ion source provides sufficient storage stability for a commercial toothpaste.

## Claims

### Claims for the following Contracting States : AT, DE, DK, LU, NL, SE

1. A toothpaste composition which comprises: an anti-calculus agent comprising at least 4% by weight, based on the total weight of the toothpaste, of a water-soluble alkali metal tripolyphosphate salt; a dental abrasive other than calcium pyrophosphate; and an orally acceptable vehicle; the toothpaste having a pH of from 8 to 10.

2. A composition as claimed in claim 1 which further comprises a substantially water insoluble noncationic anti-bacterial agent.

3. A composition as claimed in claims 1 or 2 in which the alkali metal tripolyphosphate salt is present from 4% to 6% by weight of the composition.

4. A composition as claimed in claim 2 or claim 3 in which the pH of the composition is from 8 to 9.

5. A composition as claimed in any one of claims 2 to 4 in which the noncationic anti-bacterial agent is present in from 0.01 to 2.0% by weight of the composition.

6. A composition as claimed in any one of claims 2 to 5 in which the noncationic anti-bacterial agent is selected from a diphenyl ether of the formula (I):

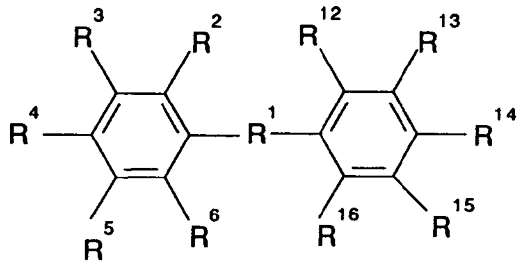

(I)

in which $R^1$ is oxygen, sulphur, or an alkylene group of from one to six carbon atoms and each of $R^2$ to $R^6$ and $R^{12}$ to $R^{16}$ is hydrogen, hydroxyl or a halogen; a phenolic or bisphenolic compound; a benzoate ester or a halogenated carbanilide.

7. A composition as claimed in claim 6 in which the compound of formula (I) is selected from 5,5'-dichloro-2,2'-dihydroxydiphenylmethane;
2,2'-dihydroxy-3,5,6,3',5',6'-hexachlorodiphenylmethane,
3,3'-dibromo-5,5'-dichloro-2,2'-dihydroxydiphenylether, or
2,4,4'-trichloro-2'-hydroxy- diphenylether.

8. A composition as claimed in any one of claims 1 to 7 in which the abrasive is selected from silica, plastics particles, alumina, calcium carbonate or zinc orthophosphate.

9. A composition as claimed in any one of claims 1 to 8 further having a phosphatase enzyme inhibitor comprising a fluoride ion source.

10. A composition as claimed in claim 9 in which the fluoride ion source is an alkali metal fluoride.

11. A composition as defined in any one of the preceding claims for use in inhibiting dental calculus.

12. A composition as defined in claim 2 for use in inhibiting dental calculus and dental plaque.

**Claims for the following Contracting State : ES**

1. A process for preparing a toothpaste composition which comprises: an anti-calculus agent comprising at least 4% by weight, based on the total weight of the toothpaste, of a water-soluble alkali metal tripolyphosphate salt; a dental abrasive other than calcium pyrophosphate; and an orally acceptable vehicle; the toothpaste having a pH of from 8 to 10; which process comprises admixing the ingredients in the appropriate quantities and if necessary or desired, adjusting the pH.

2. A process for preparing a composition as claimed in Claim 1 which further comprises a substantially water insoluble noncationic anti-bacterial agent, which process comprises admixing the ingredients in the appropriate quantities and if necessary or desired, adjusting the pH.

3. A process for preparing a composition as claimed in claim 2 in which the alkali metal tripolyphosphate salt is present from 4% to 6% by weight of the composition.

4. A process for preparing a composition as claimed in claim 2 or claim 3 in which the pH of the composition is from 8 to 9.

5. A process for preparing a composition as claimed in any one of claims 2 to 4 in which the noncationic antibacterial agent is present in from 0.01 to 2.0% by weight of the composition.

6. A process for preparing a composition as claimed in any one of claims 2 to 6 in which the noncationic antibacterial agent is selected from a diphenyl ether of the formula (I):

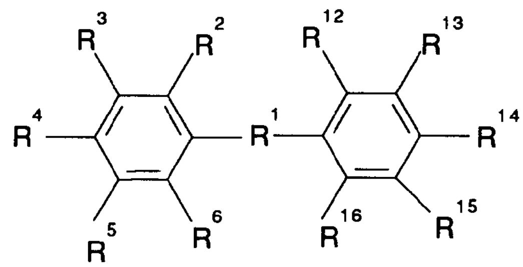

(I)

in which $R^1$ is oxygen, sulphur, or an alkylene group of from one to six carbon atoms and each of $R^2$ to $R^6$ and $R^{12}$ to $R^{16}$ is hydrogen, hydroxyl or a halogen; a phenolic or bisphenolic compound; a benzoate ester or a halogenated carbanilide.

7. A process for preparing a composition as claimed in claim 6 in which the compound of formula (I) is selected from
5,5'-dichloro-2,2'-dihydroxydiphenylmethane;
2,2'-dihydroxy-3,5,6,3',5',6'-hexachlorodiphenylmethane,
3,3'-dibromo-5,5'-dichloro-2,2'- dihydroxydiphenylether, or
2,4,4'-trichloro-2'-hydroxy- diphenylether.

8. A process for preparing a composition as claimed in any one or claims 1 to 7 in which the abrasive is selected from silica, plastics particles, alumina, calcium carbonate or zinc orthophosphate.

9. A process for preparing a composition as claimed in any one of claims 1 to 8 in which the composition further comprises a phosphatase enzyme inhibitor comprising a fluoride ion source.

10. A process for preparing a composition as claimed in claim 9 in which the the fluoride ion source is an alkali metal fluoride.

11. A composition as defined in any one of claims 1 to 10 for use in inhibiting dental calculus.

12. A composition as defined in claim 2 for use in inhibiting dental calculus and dental plaque.

**Patentansprüche**

**Patentansprüche für folgende Vertragsstaaten : AT, DE, DK, LU, NL, SE**

1. Zahnpasta-Zusammensetzung mit einem gegen Zahnsteinbildung wirkenden Agens, umfassend mindestens 4 Gew.-% eines wasserlöslichen Alkalimetalltripolyphosphats, bezogen auf das Gesamtgewicht der Zahnpasta, einem Schleifmittel ausgenommen Calciumpyrophosphat und einem im Mund verträglichen Vehicel, wobei die Zahnpasta einen pH-Wert von 8 bis 10 hat.

2. Zusammmensetzung nach Anspruch 1, die außerdem einen im wesentlichen wasserunlöslichen, nicht-kationischen, antibakteriellen Wirkstoff umfaßt.

3. Zusammensetzung nach Anspruch 1 oder 2, wobei das Alkalimetalltripolyphosphats in einer Menge von 4 bis 6 Gew.-%, bezogen auf die Zusammensetzung, vorliegt.

4. Zusammensetzung nach Anspruch 2 oder 3, wobei die Zusammensetzung einen pH-Wert von 8 bis 9 hat.

5. Zusammensetzung nach einem der Ansprüche 2 bis 4, wobei der nicht-kationische, antibakterielle Wirkstoff in einer Menge von 0.01 bis 2 Gew.-%, bezogen auf die Zusammensetzung, vorliegt.

6. Zusammensetzung nach einem der Ansprüche 2 bis 5, wobei der nicht-kationische, antibakterielle Wirkstoff ausgewählt ist aus einem Diphenylether der Formel (I):

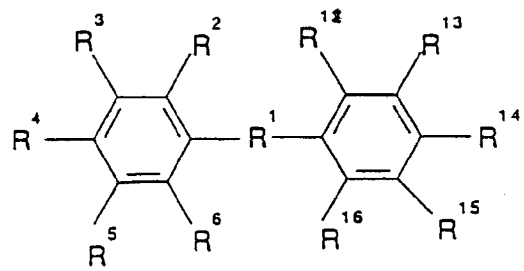

(I)

wobei der Rest $R^1$ Sauerstoff, Schwefel oder ein Alkylenrest mit 1 bis 6 C-Atomen ist und jeder der Reste $R^2$ bis $R^6$ und $R^{12}$ bis $R^{16}$ Wasserstoff, Hydroxyl oder ein Halogen, eine phenolische oder bisphenolische Verbindung, ein Benzoatester oder ein halogeniertes Carbanilid ist.

7. Zusammensetzung nach Anspruch 6, wobei die Verbindung der Formel (I) ausgewählt ist aus 5,5'-Dichlor-2,2'-dihydroxydiphenylmethan, 2,2'-Dihydroxy-3,5,6,3',5',6'-hexachlordiphenylmethan, 3,3'-Dibrom-5,5'-dichlor-2,2'-dihydroxydiphenylether oder 2,4,4'-Trichlor-2'-hydroxydiphenylether.

8. Zusammensetzung nach einem der Ansprüche 1 bis 7, wobei das Schleifmittel ausgewählt ist aus Siliciumdioxid, Kunststoffteilchen, Aluminiumoxid, Calciumcarbonat oder Zinkorthophosphat.

9. Zusammensetzung nach einem der Ansprüche 1 bis 8, die außerdem einen Phosphatase-Enzym-Inhibitor enthält, der eine Fluoridionenquelle umfaßt.

10. Zusammensetzung nach Anspruch 9, wobei die Fluoridionenquelle ein Alkalimetallfluorid ist.

11. Zusammensetzung nach einem der vorhergehenden Ansprüche zur Hemmung der Zahnsteinbildung.

12. Zusammensetzung nach Anspruch 2 zur Hemmung der Zahnsteinbildung und des Zahnbelags.

**Patentansprüche für folgenden Vertragsstaat : ES**

1. Verfahren zur Herstellung einer Zahnpasta-Zusammensetzung, die ein gegen Zahnsteinbildung wirkendes Agens, umfassend, mindestens 4 Gew.-% eines wasserlöslichen Alkalimetalltripolyphosphats, bezogen auf das Gesamtgewicht der Zahnpasta, ein Zahn-Schleifmittel ausgenommen Calciumpyrophosphat und ein im Mund verträgliches Vehicel umfaßt, wobei die Zahnpasta einen pH-Wert von 8 bis 10 hat, dadurch gekennzeichnet, daß man die Bestandteile in den geeigneten Mengen miteinander vermischt und gegebenenfalls den pH-Wert einstellt.

2. Verfahren zur Herstellung einer Zusammensetzung nach Anspruch 1, das weiterhin einen im wesentlichen wasserunlöslichen, nicht-kationischen, antibakteriellen Wirkstoff umfaßt, dadurch gekennzeichnet, daß man die Bestandteile in den geeigneten Mengen miteinander vermischt und gegebenenfalls den pH-Wert einstellt

3. Verfahren zur Herstellung einer Zusammensetzung nach Anspruch 2, wobei das Alkalimetalltripolyphosphats in einer Menge von 4 bis 6 Gew.-%, bezogen auf die Zusammensetzung, vorliegt.

4. Verfahren zur Herstellung einer Zusammensetzung nach Anspruch 2 oder 3, wobei die Zusammensetzung einen pH-Wert von 8 bis 9 hat.

5. Verfahren zur Herstellung einer Zusammensetzung nach einem der Ansprüche 2 bis 4, wobei der nicht-kationische, antibakterielle Wirkstoff in einer Menge von 0.01 bis 2 Gew.-%, bezogen auf die Zusammensetzung, vorliegt.

6. Verfahren zur Herstellung einer Zusammensetzung nach einem der Ansprüche 2 bis 5, wobei der nicht-kationische, antibakterielle Wirkstoff ausgewählt ist aus einem Diphenylether der Formel (I):

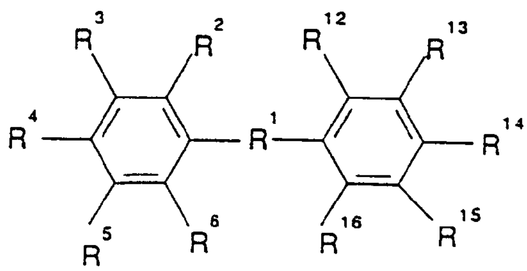

(I)

in der der Rest $R^1$ Sauerstoff, Schwefel oder ein Alkylenrest mit 1 bis 6 C-Atomen ist und jeder der Reste $R^2$ bis $R^6$ und $R^{12}$ bis $R^{16}$ Wasserstoff, Hydroxyl oder ein Halogen, eine phenolische oder bisphenolische Verbindung, ein Benzoatester oder ein halogeniertes Carbanilid ist.

7. Verfahren zur Herstellung einer Zusammensetzung nach Anspruch 6, wobei die Verbindung aus Formel (I) ausgewählt ist aus 5,5'-Dichlor-2,2'-dihydroxydiphenylmethan, 2,2'-Dihydroxy-3,5,6,3',5',6'-Hexachlordiphenylmethan, 3,3'-Dibrom-5,5'-dichlor-2,2'-dihydroxydiphenylether oder 2,4,4'-Trichlor-2'-hydroxydiphenylether.

8. Verfahren zur Herstellung einer Zusammensetzung nach einem der Ansprüche 1 bis 7, wobei das Schleifmittel ausgewählt ist aus Siliciumdioxid, Kunststoffteilchen, Aluminiumoxid, Calciumcarbonat oder Zinkorthophosphat.

9. Verfahren zur Herstellung einer Zusammensetzung nach einem der Ansprüche 1 bis 8, wobei die Zusammensetzung außerdem einen Phosphatase-Enzym-Inhibitor umfaßt, der eine Fluoridionenquelle umfaßt.

10. Verfahren zur Herstellung einer Zusammensetzung nach Anspruch 9, wobei die Fluoridionenquelle ein Alkalimetallfluorid ist.

11. Zusammensetzung nach einem der Ansprüche 1 bis 10 zur Hemmung der Zahnsteinbildung.

12. Zusammensetzung nach Anspruch 2 zur Hemmung der Zahnsteinbildung und des Zahnbelags.

**Revendications**

**Revendications pour les Etats contractants suivants : AT, DE, DK, LU, NL, SE**

1. Composition de dentifrice comprenant : un agent anti-tartre comprenant au moins 4 % en poids, sur la base du poids total du dentifrice, d'un sel tripolyphosphate de métal alcalin soluble dans l'eau ; un abrasif dentaire autre que le pyrophosphate de calcium ; et un véhicule oralement acceptable ; le dentifice ayant un pH de 8 à 10.

2. Composition selon la revendication 1, comprenant, en outre, un agent antibactérien non cationique essentiellement insoluble dans l'eau.

3. Composition selon la revendication 1 ou 2, dans laquelle le sel tripolyphosphate de métal alcalin est présent en une quantité valant de 4 % à 6 % en poids de la composition.

4. Composition selon la revendication 2 ou 3, dans lequel le pH de la composition est de 8 à 9.

5. Composition selon une quelconque des revendications 2 à 4, dans laquelle l'agent antibactérien non cationique est présent en une quantité valant de 0,01 à 2,0% en poids de la composition.

6. Composition selon une quelconque des revendications 2 à 5, dans laquelle l'agent antibactérien non cationique est choisi dans le groupe comprenant un éther diphénylique de formule (I) :

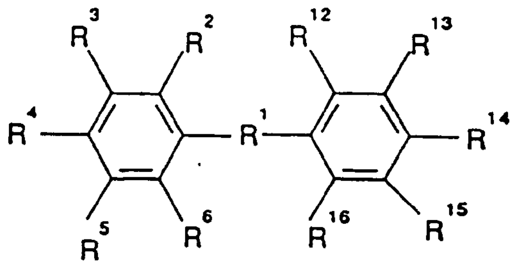

(I)

dans laquelle $R^1$ représente l'oxygène, le soufre ou un groupe alkylène comportant de 1 à 6 atomes de carbone et chaque $R^2$ à $R^6$ et $R^{12}$ à $R^{16}$ représente un atome d'hydrogène ou d'halogène ou un groupe hydroxyle ;
un composé phénolique ou bisphénolique ; un ester benzoate ou un carbanilide halogéné.

7. Composition selon la revendication 6, dans laquelle le composé de formule (I) est choisi dans le groupe comprenant :
le 5,5'-dichloro-2,2'-dihydroxydiphénylméthane,
le 2,2'-dihydroxy-3,5,6,3',5',6'-hexachlorodiphénylméthane,
l'éther 3,3'-dibromo-5,5'-dichloro-2,2'-dihydroxydiphénylique ou l'éther 2,4,4'-trichloro-2'-hydroxydiphénylique.

8. Composition selon une quelconque des revendications 1 à 7, dans laquelle l'abrasif est choisi dans le groupe comprenant la silice, des particules de matière plastique, l'alumine, le carbonate de calcium ou l'orthophosphate de zinc.

9. Composition selon une quelconque des revendications 1 à 8, comprenant, en outre, un inhibiteur d'enzyme phosphatase, comprenant une source d'ion fluorure.

10. Composition selon la revendication 9, dans laquelle la source d'ion fluorure est un fluorure de métal alcalin.

11. Composition telle que définie dans une quelconque des revendications précédentes, à utiliser pour empêcher la formation du tartre.

12. Composition telle que définie dans la revendication 2, à utiliser pour empêcher la formation du tartre et de la plaque dentaire.

**Revendications pour l'Etat contractant suivant : ES**

1. Procédé de préparation d'une composition de dentifrice comprenant : un agent anti-tartre comprenant au moins 4 % en poids, sur la base du poids total du dentifrice, d'un sel tripolyphosphate de métal alcalin soluble dans l'eau ; un abrasif dentaire autre que le pyrophosphate de calcium ; et un véhicule oralement acceptable ; le dentifice ayant un pH de 8 à 10, lequel procédé comprend le mélange des ingrédients en les quantités appropriées et, si cela est nécessaire, ou si on le désire, l'ajustement du pH.

2. Procédé de préparation d'une composition selon la revendication 1, comprenant, en outre, un agent antibactérien non cationique essentiellement insoluble dans l'eau, lequel procédé comprend le mélange des ingrédients en les quantités appropriées et, si cela est nécessaire ou si on le désire, l'ajustement du pH.

3. Procédé de préparation d'une composition selon la revendication 2, dans lequel le sel tripolyphosphate de métal alcalin est présent en une quantité valant de 4 % à 6 % en poids de la composition.

4. Procédé de préparation d'une composition selon la revendication 2 ou 3, dans lequel le pH de la composition est de 8 à 9.

5. Procédé de préparation d'une composition selon une quelconque des revendications 2 à 4, dans lequel l'agent antibactérien non cationique est présent en une quantité valant de 0,01 à 2,0 % en poids de la composition.

6. Procédé de préparation d'une composition selon une quelconque des revendications 2 à 5, dans lequel l'agent antibactérien non cationique est choisi dans le groupe comprenant un éther diphénylique de formule (I) :

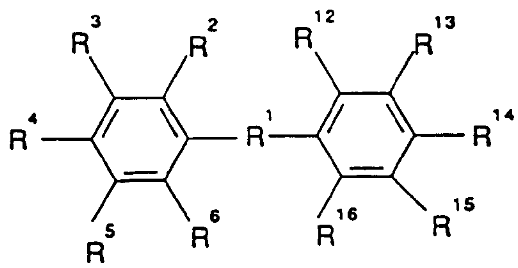

(I)

dans laquelle $R^1$ représente l'oxygène, le soufre ou un groupe alkylène comportant de 1 à 6 atomes de carbone et chaque $R^2$ à $R^6$ et $R^{12}$ à $R^{16}$ représente un atome d'hydrogène ou d'halogène ou un groupe hydroxyle ;
un composé phénolique ou bisphénolique ; un ester benzoate ou un carbanilide halogéné.

7. Procédé de préparation d'une composition selon la revendication 6, dans lequel le composé de formule (I) est choisi dans le groupe comprenant :
le 5,5'-dichloro-2,2'-dihydroxydiphénylméthane,
le 2,2'-dihydroxy-3,5,6,3',5',6'-hexachlorodiphénylméthane,
l'éther 3,3'-dibromo-5, 5'-dichloro-2,2'-dihydroxydiphénylique ou l'éther 2,4,4'-trichloro-2'-hydroxydiphénylique.

8. Procédé de préparation d'une composition selon une quelconque des revendications 1 à 7, dans lequel l'abrasif est choisi dans le groupe comprenant la silice, des particules de matière plastique, l'alumine, le carbonate de calcium ou l'orthophosphate de zinc.

9. Procédé de préparation d'une composition selon une quelconque des revendications 1 à 8, dans lequel la composition contient, en outre, un inhibiteur d'enzyme phosphatase, comprenant une source d'ion fluorure.

10. Procédé de préparation d'une composition selon la revendication 9, dans lequel la source d'ion fluorure est un fluorure de métal alcalin.

11. Composition telle que définie dans une quelconque des revendications 1 à 10, à utiliser pour empêcher la formation du tartre.

12. Composition telle que définie dans la revendication 2, à utiliser pour empêcher la formation du tartre et de la plaque dentaire.